(19) Europäisches Patentamt
European Patent Office
Office européen des brevets

(11) **EP 4 664 308 A1**

(12) **EUROPEAN PATENT APPLICATION**
published in accordance with Art. 153(4) EPC

(43) Date of publication:
**17.12.2025 Bulletin 2025/51**

(21) Application number: **24769643.8**

(22) Date of filing: **15.01.2024**

(51) International Patent Classification (IPC):
**G06F 16/174** (2019.01)

(52) Cooperative Patent Classification (CPC):
**G06F 3/06; G06F 16/172; G06F 16/174; H03M 7/30**

(86) International application number:
**PCT/CN2024/072222**

(87) International publication number:
**WO 2024/187947 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.03.2023 CN 202310303465**

(71) Applicant: **Huawei Technologies Co., Ltd.**
**Shenzhen, Guangdong 518129 (CN)**

(72) Inventors:
• **DONG, Zheting**
**Shenzhen, Guangdong 518129 (CN)**
• **QIN, Liang**
**Shenzhen, Guangdong 518129 (CN)**
• **SUN, Zhaoyi**
**Shenzhen, Guangdong 518129 (CN)**
• **SUN, Jie**
**Shenzhen, Guangdong 518129 (CN)**

(74) Representative: **MERH-IP Matias Erny Reichl Hoffmann**
**Patentanwälte PartG mbB**
**Paul-Heyse-Straße 29**
**80336 München (DE)**

(54) **DATA PROCESSING METHOD AND RELATED DEVICE**

(57) This application discloses a data processing method and a related device. A file header including block information, a description information compression parameter, a sequence compression parameter, and a quality score compression parameter is obtained. A plurality of blocks are obtained based on the block information and a first file. Data corresponding to each of the plurality of blocks is compressed based on the compression parameters in the file header to obtain a data block corresponding to each of the plurality of blocks. A second file is obtained, where the second file includes the file header and the data block corresponding to each of the plurality of blocks. It can be learned that a compressed file format is defined, a compression scheme-related parameter is preconfigured in a file header of a file conforming to the defined compressed file format, the file is divided into blocks and compressed according to an indication of the file header, a compression result is inserted in a data block after the file header in a manner conforming to the defined compressed file format, to obtain a compressed file. In this way, efficient data compression can be implemented.

FIG. 9

## Description

This application claims priority to Chinese Patent Application No. 202310303465.3, filed with the China National Intellectual Property Administration on March 13, 2023 and entitled "DATA PROCESSING METHOD AND RELATED DEVICE", which is incorporated herein by reference in its entirety.

## TECHNICAL FIELD

This application relates to the field of storage technologies, and in particular, to a data processing method and a related device.

## BACKGROUND

Facing rapid growth of data, an enterprise needs to continuously purchase a large quantity of storage devices to meet an increasing storage requirement. However, simply increasing a storage capacity is not likely to resolve the problem fundamentally. In view of this, a concept of efficient storage is proposed to alleviate a space growth issue of a storage system, reduce space occupied by data, simplify storage management, maximize utilization of existing resources, and reduce costs. In a data compression technology, data is recoded to reduce redundancy and a data capacity. The data compression technology is one of key technologies for data reduction.

A current data compression technology cannot meet requirements in some scenarios. For example, with massive application of gene data, during compression of a gene sequencing file, massive parameters of a model used for compression need to be recorded, a plurality of models used for compression need to be maintained, or bit-level dynamic encoding needs to be performed. Consequently, large storage space is occupied, a large quantity of computing resources are used, processing takes a long time, and compression efficiency is low.

Based on this, a more appropriate data processing method urgently needs to be provided to overcome low compression efficiency of the current data compression technology.

## SUMMARY

Based on this, this application provides a data processing method and a related device, to quickly compress to-be-compressed data at a high compression ratio, and make storage of the to-be-compressed data easier.

According to a first aspect, this application provides a data processing method. The method may include: obtaining a file header, where the file header includes block information, a description information compression parameter, a sequence compression parameter, and a quality score compression parameter; obtaining a plurality of blocks based on the block information and a first file, where each of the plurality of blocks includes first description information, a first sequence, and a first quality score; compressing the first description information, the first sequence, and the first quality score in each of the plurality of blocks based on the description information compression parameter, the sequence compression parameter, and the quality score compression parameter to obtain a data block corresponding to each of the plurality of blocks, where the data block includes a description information compression result, a sequence compression result, and a quality score compression result of a corresponding block; and obtaining a second file, where the second file is a file obtained by compressing the first file, and the second file includes the file header and the data block corresponding to each of the plurality of blocks. In this way, a compressed file format is defined, a compression scheme-related parameter is preconfigured in a file header of a file conforming to the defined compressed file format, the file is divided into blocks and compressed according to an indication of the file header, a compression result is inserted in a data block after the file header in a manner conforming to the defined compressed file format, to obtain a compressed file. In this way, efficient data compression can be implemented.

In a possible implementation, obtaining the plurality of blocks based on the block information and the first file may include: performing block division on the first file based on the block information to obtain a plurality of data entry sets, where each of the plurality of data entry sets includes at least one data entry; and classifying a data entry in any one of the plurality of data entry sets based on a data type to obtain a block corresponding to the any data entry set, where the data type includes at least one of description information, a sequence, and a quality score.

In an example, the block information includes a memory size identifier (memID). In this case, for example, performing block division on the first file based on the block information to obtain the plurality of data entry sets may include: determining memory information based on the memID; and performing block division on the first file based on the memory information to obtain the plurality of data entry sets. The memory information may be an upper memory limit or a memory size of each block.

In an example, the any data entry set includes a first data entry and a second data entry, the first data entry includes second description information, a second sequence, and a second quality score, and the second data entry includes third description information, a third sequence, and a third quality score. Therefore, in the block corresponding to the any data entry set, the first description information includes the second description information and the third description information, the first sequence includes the second sequence and the third sequence, and the first quality score includes the second quality score and the third quality score.

In a possible implementation, the block corresponding to the any data entry set may further include length information of each data entry in the any data entry set, the length information is a length of a sequence or a quality score in a corresponding data entry, and the data block further includes data obtained by compressing length information of each data entry in the corresponding block. In this way, a necessary basis is provided for subsequent decompression of the second file.

In a possible implementation, the quality score compression parameter includes a first parameter and a second parameter, the first parameter indicates a preceding-context model, the preceding-context model is used to determine a feature value of a preceding-context string of a to-be-encoded character, and the second parameter is used to determine a compression model corresponding to the feature value.

In an example, compressing the first quality score in each of the plurality of blocks based on the quality score compression parameter may include: for any one of a plurality of quality score characters in any one of the plurality of blocks, obtaining a preceding-context string of the any quality score character; determining a feature value of the any quality score character based on the first parameter and the preceding-context string of the any quality score character; determining a target compression model based on the second parameter and the feature value of the any quality score character; compressing the any quality score character based on the target compression model; and obtaining, based on compression results of the plurality of quality score characters, a compression result of the first quality score corresponding to the any block. In this way, dynamic compression is performed per quality score character. Because feature values of all quality score characters have been centrally pre-calculated, although a historically used target compression model needs to be continuously maintained and a historical feature value corresponding to a previous same feature value needs to be updated by using a most recently used target compression model, causing high pressure on a memory and processing, efficiency is still improved compared with a current compression scheme.

Determining the target compression model based on the second parameter and the feature value of the any quality score character may include: sorting quality score characters in the any block based on the feature value to obtain a plurality of quality score strings, where each of the plurality of quality score strings corresponds to a same feature value; and for a first quality score string among the plurality of quality score strings, determining a first compression model based on a feature value corresponding to the first quality score string. Therefore, compressing the any quality score character based on the target compression model may include: compressing the first quality score string based on the first compression model. In this way, quality score characters are classified based on feature values, to reduce a quantity of times that a compression process is performed. In addition, after a quality score string corresponding to each feature value is compressed, a target compression model corresponding to the feature value may not be maintained in the memory, so that fast and efficient "static" encoding can be implemented, and pressure on the memory and processing is also low.

In an example, in a technical solution of "static" encoding, to effectively restore the first file, the second file may further include a feature value of the 1st quality score character in the any block.

In a possible implementation, the quality score compression parameter further includes a third parameter, the third parameter indicates a threshold of a string length for performing a compression operation, and the method may further include: storing, in a target area, a quality score string with a length less than the threshold of a string length among the plurality of quality score strings; and compressing the quality score string in the target area based on a general-purpose compression algorithm. This avoids a plurality of times of separate compression on short quality score strings. These short quality score characters are accumulated to the target area, and are centrally compressed by using the general-purpose compression algorithm, to effectively improve compression efficiency.

In a possible implementation, the description information compression parameter may include a partitioning strategy and a second compression model. In this case, compressing the first description information in each of the plurality of blocks based on the description information compression parameter may include: partitioning first description information in any one of the plurality of blocks according to the partitioning strategy to obtain a plurality of description segments; and for any one of the plurality of description segments, performing the following steps based on the second compression model: if the any description segment is integer-type data, converting the any description segment into a binary format and then compressing the any description segment; or if the any description segment is not integer-type data, compressing the any description segment by using an LZ algorithm; and obtaining, based on compression results corresponding to the plurality of description segments, the description information compression result corresponding to the any block. In this way, description information in each block is quickly compressed.

In a possible implementation, the sequence compression parameter may include a packing length and a third compression model. In this case, compressing the first sequence in each of the plurality of blocks based on the sequence compression parameter may include: processing, according to an N-pack strategy, a first sequence in any one of the plurality of blocks to obtain a fourth sequence, where N is an integer greater than 1, and N matches the packing length; and compressing the fourth sequence based on the third compression model to obtain the sequence compression result corresponding to the any block. In this way, a sequence in each block is quickly compressed.

In a possible implementation, the description information compression result may include a size of compressed description information and a first compressed stream corresponding to description information, where the first compressed stream includes data obtained by compressing the description information; the sequence compression result may include a size of a compressed sequence and a second compressed stream corresponding to a sequence, where the second compressed stream includes data obtained by compressing the sequence; and the quality score compression result may include a size of a compressed quality score and a third compressed stream corresponding to a quality score, where the third compressed stream includes data obtained by compressing the quality score. In addition, the description information compression result may further include a size of uncompressed description information, to enable a user to learn of a size of the part after decompression, and provide a reference for the user to perform decompression. Similarly, the sequence compression result may further include a size of an uncompressed sequence, and the quality score compression result may further include a size of an uncompressed quality score.

In a possible implementation, the file header further includes at least one of the following fields: a format signature, a file size, a third-line status, or a check bit.

In a possible implementation, the second file further includes a directory block, and the target block indicates a sequence number of the 1st data entry in a block, a position of the block in the second file, and a position of the block in the first file. In this way, it is convenient for the user to randomly read a part of data in the second file.

In a possible implementation, the first file is a FASTQ file.

In a possible implementation, the method may further include: decompressing, based on the file header of the second file, a plurality of data blocks in the second file to obtain the first file. In this way, an efficiently compressed file is effectively decompressed, to provide a basis for implementing friendly file storage.

According to a second aspect, this application further provides a data processing method. The method may include: obtaining, based on a preceding-context model, feature values of a plurality of characters included in first data, where a feature value of any one of the plurality of characters represents a feature of a preceding-context string, with a preset length, of the any character; determining a target compression model based on the feature value of the any character; and compressing the any character based on the target compression model to obtain second data, where the second data is a compression result of the first data. In this way, efficient compression is implemented based on a higher-order model, and less storage space is occupied.

In a possible implementation, obtaining, based on the preceding-context model, the feature values of the plurality of characters included in the first data may include: for any one of the plurality of characters, obtaining a preceding-context string, with a preset length, of the any character; and determining a feature value of the any character based on a length of each compressed preceding-context character in the preceding-context string, each preceding-context character in the preceding-context string, and a preset calculation rule.

In a possible implementation, determining the target compression model based on the feature value of the any character may include: sorting a plurality of characters in the first data based on the feature value to obtain a plurality of strings, where each of the plurality of strings corresponds to a same feature value; and for the 1st one of the plurality of strings, determining a first compression model based on a mapping relationship and a feature value corresponding to the 1st string, where the mapping relationship includes a correspondence between a plurality of groups of feature values and compression models. In this case, compressing the any character based on the target compression model to obtain the second data may include: compressing the 1st string based on the first compression model to obtain third data, where the second data includes the third data.

The first data may be a quality score in any block obtained by dividing a FASTQ file into blocks.

According to a third aspect, this application provides a communication apparatus. The communication apparatus includes a processing unit. The processing unit is configured to implement the methods corresponding to the first aspect, the second aspect, and the possible implementations of the first aspect and the second aspect.

According to a fourth aspect, this application provides a communication device. The communication device includes a processor and a memory. The processor is configured to execute instructions stored in the memory, to enable the communication device to implement the methods corresponding to the first aspect, the second aspect, and the possible implementations of the first aspect and the second aspect.

According to a fifth aspect, this application further provides a storage medium. The storage medium stores instructions. When the instructions are run on a processor, the methods corresponding to the first aspect, the second aspect, and the possible implementations of the first aspect and the second aspect are implemented.

According to a sixth aspect, this application further provides a program product. The program product includes a program. When the program is run on a processor, the methods corresponding to the first aspect, the second aspect, and the possible implementations of the first aspect and the second aspect are implemented.

According to a seventh aspect, this application provides a chip, including a memory and a processor. The memory is configured to store instructions. The processor is configured to invoke the instructions from the memory and run the instructions, to implement the methods corresponding to the first aspect, the second aspect, and the possible implementations of the first aspect and the second aspect.

**BRIEF DESCRIPTION OF DRAWINGS**

FIG. 1 is a diagram of a file obtained by compressing a FASTQ file according to an embodiment of this application;

FIG. 2 is a diagram of a file header in a file shown in FIG. 1;

FIG. 3 is a diagram of a quality score compression parameter in a file header shown in FIG. 2;

FIG. 4 is a diagram of a data block in a file shown in FIG. 1;

FIG. 5 is a diagram of compressed quality scores in a data block shown in FIG. 4;

FIG. 6 is a diagram of an ending block in a file shown in FIG. 1;

FIG. 7 is a diagram of an optional block in a file shown in FIG. 1;

FIG. 8 is a diagram of a directory area included in a file shown in FIG. 1;

FIG. 9 is a schematic flowchart of a data processing method 100 according to an embodiment of this application;

FIG. 10 is a schematic flowchart of an implementation of S1031 according to an embodiment of this application;

FIG. 11 is a schematic flowchart of a data processing method 200 according to an embodiment of this application;

FIG. 12 is a diagram of a structure of a communication apparatus 1200 according to an embodiment of this application;

FIG. 13 is a diagram of a structure of a communication apparatus 1300 according to an embodiment of this application; and

FIG. 14 is a diagram of a structure of a communication device 1400 according to an embodiment of this application.

**DESCRIPTION OF EMBODIMENTS**

With rapid growth of data, a data compression technology becomes necessary and important in data transmission and storage. In a current data compression technology, lossless compression can be performed on data in various scenarios. However, in some scenarios, efficient data compression cannot be implemented by using current conventional data compression technologies. This is an unacceptable disadvantage for a field including a large amount of data. For example, with massive application of gene data, compression efficiency achieved by compressing a FASTQ file, generated through gene sequencing, as a common to-be-compressed file only by using a conventional data compression technology cannot meet a requirement of the field. For example, fqzcomp and gtz are two conventional compression algorithms. However, in fqzcomp, a model including a large quantity of parameters needs to be maintained during compression of a FASTQ file. On a communication apparatus, a huge model is represented as a data structure that occupies a large amount of memory. Maintaining a huge data structure in a performance-limited system greatly affects operation efficiency, leading to quite low compression efficiency. gtz is a bit-level dynamic encoding algorithm in which bits are encoded one by one and a plurality of models that may be needed by characters in subsequent bits need to be maintained, leading to low compression efficiency.

Based on this, embodiments of this application provide a data processing method. In the method, a compression parameter and block information are preconfigured in a file header. During compression of a to-be-compressed first file, block division may be first performed on the first file based on the block information to obtain a plurality of blocks. Then each block is compressed based on the compression parameter to obtain a corresponding data block. Then a second file is obtained based on the file header and data blocks corresponding to the plurality of blocks of the first file, where the second file is a file obtained by compressing the first file according to the method provided in embodiments of this application. It can be learned that, in embodiments of this application, a compressed file format is defined, a compression scheme-related parameter is preconfigured in a file header of a file conforming to the defined compressed file format, the file is divided into blocks and compressed according to an indication of the file header, a compression result is inserted in a data block after the file header in a manner conforming to the defined compressed file format, to obtain a compressed file. In this way, efficient data compression can be implemented.

The method provided in embodiments of this application may be applied to unstructured data storage, to reduce storage capacity expansion and reduce storage costs. For example, a controller, a cloud, or a server may provide a program product corresponding to the method in embodiments of this application. A communication apparatus may download the program product from the controller, the cloud, or the server, and use the program product. The communication apparatus may integrate a functional module that can carry the program product into a compressor plug-in module of the communication apparatus. The functional module may be implemented by using software or hardware.

The data processing method provided in embodiments of this application is applicable to, for example, a FASTQ file. Embodiments of this application are described below by using compression and decompression of the FASTQ file as an example.

The FASTQ file is a file format for storing data obtained through gene sequencing. The FASTQ file may include a plurality of data entries. Each data entry may include four lines: a description information line, a base sequence line, a comment line, and a quality score line. A base in the base sequence line is in a one-to-one correspondence with a quality score in the quality score line. The quality score indicates sequencing quality of a corresponding base. The comment line may be empty, or may include a special symbol, or may include description information in the description information line. A nucleic acid sequence may include four bases: A, C, T, and G. In the FASTQ file, the bases are represented as corresponding ASCII codes. A value of the quality score may range from 0x21 (a minimum value, which is an ASCII code of "!") to 0x7e (a maximum value, which is an ASCII code of "~") of ASCII codes. For example, a FASTQ file 1 includes a data entry 1 to a data entry 100. The data entry 1 is used as an example. The data entry 1 may be represented as <a description information line 1, a base sequence line 1, a comment line 1, and a quality score line 1>.

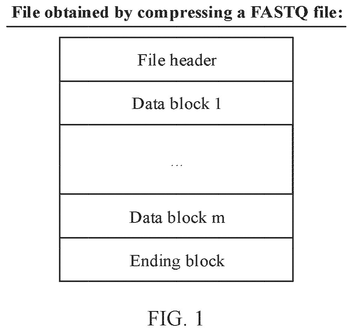

For example, a file format that is defined in embodiments of this application and that is obtained by compressing a FASTQ file may be shown in FIG. 1, and may be represented as follows: a file header, a data block 1, a data block 2, ..., a data block m, and an ending block, where m is an integer greater than 1.

The following describes specific formats of the parts in FIG. 1 by using examples.

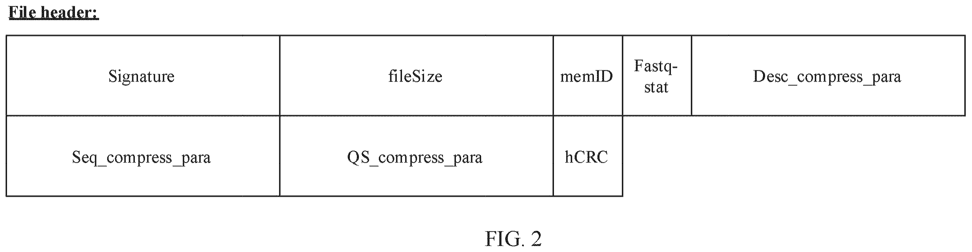

As shown in FIG. 2, the file header may include a memory size identifier (memID), a description information compression parameter (Desc _compress_para), a sequence compression parameter (Seq_compress_para), and a quality score compression parameter (QS_compress_para). Optionally, the file header may further include at least one of the following fields: a format signature (Signature), a file size (fileSize), a third-line status (Fastq-stat), or a check bit (hCRC).

The format signature is used to write a signature of the file shown in FIG. 1. The signature may be user-defined, and may be 4 bytes in length. The file size is used by a user to indicate a size of a FASTQ file obtained by decompressing the file, and may be 4 bytes in length. The memID is used to determine memory information of a block, and may be 1 byte in length. For example, a memory that is occupied by each block and that is calculated based on a value of the memID is $2^{(memID)}$ bytes. For another example, an upper memory limit of a block is calculated based on the memID, a size of a memory occupied by each block needs to be less than the upper memory limit. The third-line status may be 1 byte in length. The first 4 bits indicate a status of the $3^{rd}$ line of each data entry in the FASTQ file. For example, when the $3^{rd}$ line is in a default state, the first 4 bits of the third-line status are 0b0001; or when the $3^{rd}$ line is a repetition of content of the $1^{st}$ line, the first 4 bits of the third-line status are 0b0000. The last 4 bits indicate whether read lengths of the current FASTQ file are equal. If the read lengths are equal, the last 4 bits of the third-line status are 0b0001; or if the read lengths are not equal, the last 4 bits of the third-line status are 0b0000. The description information compression parameter may be 4 bytes in length. The $1^{st}$ byte and the $2^{nd}$ byte may indicate whether semantic word segmentation is to be performed. Values of the $1^{st}$ byte and the $2^{nd}$ byte being 1 indicates that semantic word segmentation needs to be performed. Values of the $1^{st}$ byte and the $2^{nd}$ byte being 0 indicates that semantic word segmentation does not need to be performed. The $3^{rd}$ byte and the $4^{th}$ byte indicate a compression model used for description information. For example, values of the $3^{rd}$ byte and the $4^{th}$ byte being 0 indicates that description information is to be compressed by using zstd; values of the $3^{rd}$ byte and the $4^{th}$ byte being 1 indicates that description information is to be compressed by using gzip; and when values of the $3^{rd}$ byte and the $4^{th}$ byte are 2 or values greater than 2, a corresponding compression model may be customized. The sequence compression parameter may be 4 bytes in length. The $1^{st}$ byte and the $2^{nd}$ byte may indicate whether byte-level packing is to be performed. For example, values of the $1^{st}$ byte and the $2^{nd}$ byte being 0 indicates that no packing is to be performed, values of the $1^{st}$ byte and the $2^{nd}$ byte being 1 indicates that pairwise packing is to be performed, and values of the $1^{st}$ byte and the $2^{nd}$ byte being 2 indicates that triplet packing is to be performed. The $3^{rd}$ byte and the $4^{th}$ byte may indicate a compression model used for a base sequence (referred to as a sequence below). For example, values of the $3^{rd}$ byte and the $4^{th}$ byte being 0 indicates that a sequence is to be compressed by using zstd, values of the $3^{rd}$ byte and the $4^{th}$ byte being 1 indicates that a sequence is to be compressed by using gzip, values of the $3^{rd}$ byte and the $4^{th}$ byte being 2 indicates that a sequence is to be compressed by using a first-order Markov model, values of the 3rd byte and the 4th byte being 3 indicates that a sequence is to be compressed by using a second-order Markov model, and values of the 3rd byte and the 4th byte being 4 indicates that a sequence is to be compressed by using a third-order Markov model. The hCRC may be 1 byte in length.

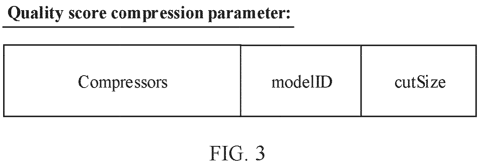

As shown in FIG. 3, the quality score compression parameter may be 4 bytes in length, and may include a compressor (Compressors) and a model identifier (modelID). Optionally, the quality score compression parameter may further include a cut size (cutSize). The Compressors may be 2 bytes in length, and indicates compression models corresponding to different values. For example, if the functional module that is in the compressor plug-in module of the communication apparatus and that is configured to carry the method provided in embodiments of this application includes a plurality of compression models, a compression model may be determined in the functional module based on a value of the Compressors. If the functional module that is in the compressor plug-in module of the communication apparatus and that is configured to carry the method provided in embodiments of this application does not include a compression model, an interface may be determined based on a value of the Compressors, and a compression model deployed in the compressor plug-in module may be invoked through the interface. The modelID indicates a preceding-context model, and may be 1 byte in length. A feature value of a preceding-context string of a to-be-encoded character may be determined based on the preceding-context model indicated by the modelID. The feature value may be used as an input value of the Compressors, to determine a corresponding compression model from the Compressors. The cutSize indicates a threshold of a string length for performing a compression operation, and a value of the cutSize varies with the compression model determined from the Compressors. The cutSize is used to adjust a balance between a compression ratio and compression efficiency, and may be 1 byte in length. The compression ratio is a percentage of a size of a compressed file to a size of an uncompressed file. The compression efficiency may be measured by using a length of time used for compression. For a same file, longer time used for compression indicates lower compression efficiency. For example, the compression efficiency may be quantized by using a compression throughput rate (or compression throughput). A higher compression throughput rate (or compression throughput) indicates higher compression efficiency.

It should be noted that, in a format of an interface of a compressor protected in embodiments of this application, the modelID and the cutSize are used as global external parameters, and all compressors compatible with the file format defined in embodiments of this application may be configured by using the following interface: Compressor(dstPtr, dstSize, srcPtr, srcSize, workSpacePtr, workSpaceSize, modelID, cutSize). The dstPtr (a start pointer of a target memory) and the dstSize (a size of the target memory) point to a compressed output memory block. The srcPtr (a start pointer of a source memory) and the srcSize (a size of the source memory) correspond to a memory in which input data is located. The workSpacePtr (a start pointer of a workspace memory) and the workSpaceSize (a size of the workspace memory) correspond to a workspace that needs to be used for data reordering in embodiments of this application. Further, the modelID and the cutSize correspond to a preceding-context model (or another higher-order model) needed in embodiments of this application.

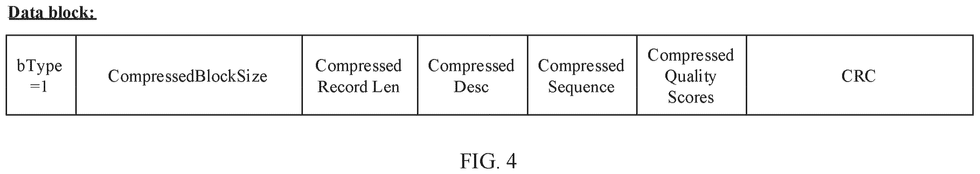

As shown in FIG. 4, the data block may include a type (bType), a compressed block size (CompersssedBlockSize), a compressed record length (Compressed Record Len), a description information compression result (Compressed Desc), a sequence compression result (Compressed Sequence), a quality score compression result (Compressed Quality Scores), and a check bit (CRC). The bType indicates a type of a block in which the bType is located, and may be 1 byte in length. bType=0 indicates that the block is a data block. bType=1 indicates that the block is an ending block. A value of bType may be extended to indicate an extended optional block. The CompersssedBlockSize indicates a size of a data block in which the CompressedBlockSize is located. The size of the data block may not include a CRC in the data block, and may be 4 bytes in length. The Compressed Record Len indicates a compression result obtained by compressing a recorded length of a sequence in each data entry in a block corresponding to the data block, and has a variable length. The Compressed Desc carries data obtained by compressing description information in the data block, and has a variable length. The Compressed Sequence carries data obtained by compressing a sequence in the data block, and has a variable length. The Compressed Quality Scores carries data obtained by compressing a quality score in the data block, and has a variable length. The CRC may be 4 bytes in length.

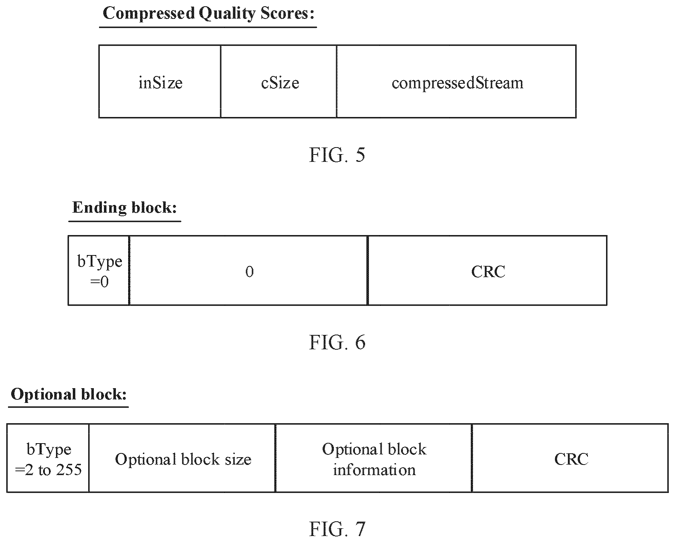

Formats of the Compressed Desc, the Compressed Sequence, and the Compressed Quality Scores in the data block may be the same. For a format of the Compressed Quality Scores, refer to FIG. 5. The format may include a size of a compressed quality score (cSize) and a compressed stream (compressedStream). The cSize may be used to determine an end of the compressedStream during decompression. The compressedStream includes at least data obtained by compressing a quality score. Optionally, the Compressed Quality Scores may further include a size of an uncompressed quality score (inSize). The inSize may indicate a size obtained after the compressed stream is decompressed, to avoid a storage risk caused by an excessively large file obtained after decompression, and provide convenience for a user.

As shown in FIG. 6, the ending block may include bType, an ending block size, and a CRC. bType=1 indicates that the block is an ending block, and the bType may be 1 byte in length. The ending block size indicates a length of the ending block. The length of the ending block may not include the CRC in the ending block. In other words, the ending block size is 0. The ending block size may be 4 bytes in length. The CRC may be 4 bytes in length.

The file format defined in embodiments of this application may further include an optional block that can be extended. A position of the optional block may be between the file header and the data block 1, or may be between the data block m and the ending block. As shown in FIG. 7, the optional block may include, for example, bType, an optional block size, optional block information, and a CRC. A value of the bType may be an integer ranging from 2 to 255. The bType indicates a type of an extended optional block, and may be 1 byte in length. The optional block size indicates a length of the optional block. The length of the optional block may not include the CRC in the optional block. The optional block size may be 4 bytes in length. The optional block information indicates content carried in the optional block, and has a variable length. The CRC may be 4 bytes in length.

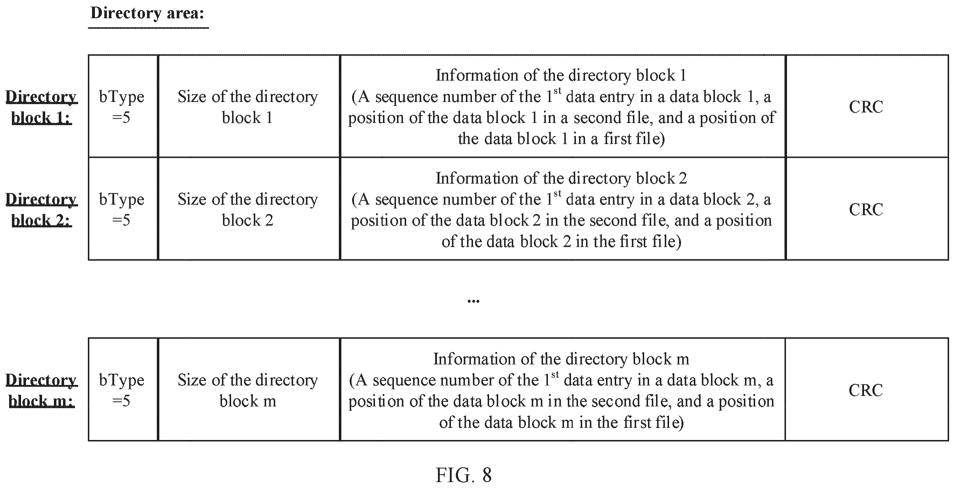

In an example, to support random reading from a FASTQ file and facilitate an operation on an ultra-large file, some optional blocks may be extended between the file header and the data block 1 as a directory area. As shown in FIG. 8, the directory area may include, for example, directory blocks whose quantity is the same as a quantity of data blocks. To be specific, the directory area includes a directory block 1 to a directory block m. For a format of each target block, refer to the format of the optional block shown in FIG. 7. A difference lies in: First, bType of the optional block is set to an undefined value, for example, bType=5, indicating that the optional block is a directory block. Second, information that can indicate a corresponding data block is inserted in optional block information. For example, the optional block information may be 20-byte content: a sequence number of the 1st data entry in the corresponding data block, a position of the corresponding data block in the file shown in FIG. 1, and a position of the corresponding data block in a FASTQ file that is compressed to obtain the file shown in FIG. 1. Lengths of the foregoing three parts in the optional block information may be sequentially 4 bytes, 8 bytes, and 8 bytes. In this way, a corresponding data block can be conveniently determined according to an indication of each directory block, and a user can flexibly and quickly read each data block in the file that is defined in embodiments of this application and that is obtained by compressing a FASTQ file.

After the file format that is defined in embodiments of this application and that is obtained by compressing a FASTQ file is described, a data processing solution applicable to the file format is described.

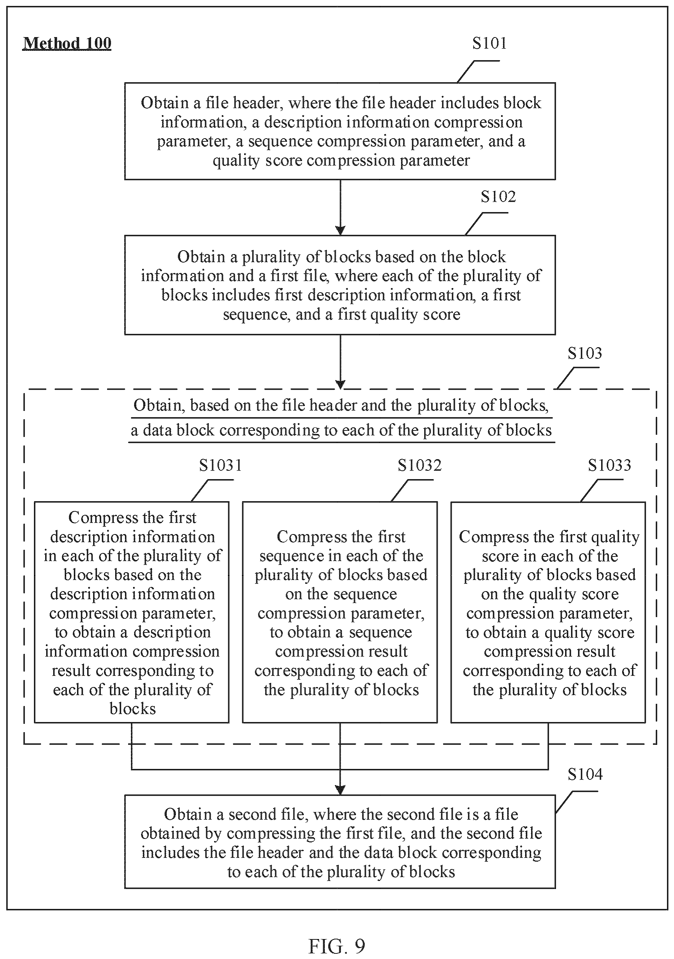

As shown in FIG. 9, a data processing method 100 provided in an embodiment of this application may include, for example, the following S101 to S104.

S101: Obtain a file header, where the file header includes block information, a description information compression parameter, a sequence compression parameter, and a quality score compression parameter.

During specific implementation, a compression-related configuration parameter may be stored in the file header, to ensure that a to-be-compressed raw file is subsequently processed based on the file header; and the file header is used as a part of a compressed file, so that the compressed file can be accurately decompressed based on a file header of the compressed file, to restore the raw file.

For related descriptions of the file header, refer to FIG. 2 and related descriptions. The block information may include a memID. The file header may further include at least one of the following fields: a format signature, a file size, a third-line status, or a check bit.

S102: Obtain a plurality of blocks based on the block information and a first file, where each of the plurality of blocks includes first description information, a first sequence, and a first quality score.

The first file is the to-be-compressed raw file, and the first file may be, for example, a FASTQ file. The foregoing storage format, defined in embodiments of this application, for lightweight compression of a FASTQ file is implemented based on separation between a block and data. To be specific, data entries in the FASTQ file are divided into different blocks, compressed based on the blocks, and then packed into a complete compressed file.

In an example, S102 may include, for example, the following steps: S1021: Perform block division on the first file based on the block information to obtain a plurality of data entry sets, where each of the plurality of data entry sets includes at least one data entry. S1022: Classify a data entry in any one of the plurality of data entry sets based on a data type to obtain a block corresponding to the any data entry set, where the data type includes at least one of description information, a sequence, and a quality score.

For S1021, if the block information in the file header includes the memID, for example, S1021 may include: first, determining memory information based on the memID; and then performing block division on the first file based on the memory information to obtain the plurality of data entries. The memory information may be a memory size or an upper memory limit of a block. For example, the memory size of the block may be determined based on the memID and a preset formula 1. The preset formula may be, for example, as follows: The memory size of the block is equal to $2^{\wedge}(\text{memID})$. For another example, the upper memory limit of the block may be determined based on the memID and a preset formula 2. In this case, during block division, it needs to be ensured that a block size does not exceed the upper memory limit.

For S1022, it is assumed that the any data entry set includes a first data entry and a second data entry, the first data entry includes second description information, a second sequence, and a second quality score, and the second data entry includes third description information, a third sequence, and a third quality score. In this case, in the block corresponding to the data entry set, the first description information includes the second description information and the third description information, the first sequence includes the second sequence and the third sequence, and the first quality score includes the second quality score and the third quality score.

During specific implementation, S1022 may include: In one aspect, description information in data entries in the data entry set is extracted to form a piece of long description information, and the long description information is used as first description information in the block corresponding to the data entry set. In another aspect, because both a sequence and a quality score in a data entry end with length information, after length information at an end of a sequence in each data entry in the data entry set is removed, two adjacent sequences are connected head to tail, to obtain a first sequence in the block corresponding to the data entry set. Similarly, after length information at an end of a quality score in each data entry in the data entry set is removed, two adjacent quality scores are connected head to tail, to obtain a first quality score in the block corresponding to the data entry set.

For example, it is assumed that the first file includes a data entry 1 to a data entry 100. S1021 is performed to divide the first file into the following five parts according to an indication of the block information: a data entry set 1 {a data entry 1, a data entry 2, ..., a data entry 20}, a data entry set 2{a data entry 21, a data entry 22, ..., a data entry 40}, a data entry set 3{a data entry 41, a data entry 42, ..., a data entry 60}, a data entry set 4{a data entry 61, a data entry 62, ..., a data entry 80}, and a data entry set 5{a data entry 81, a data entry 82, ..., a data entry 100}. S1022 is performed. The data entry set 1 is used as an example. A block 1 corresponding to the data entry set 1 may be represented as a block 1 {description information A1, a sequence B1, and a quality score C1}. The description information A1 includes description information 1, description information 2, ..., and description information 20. The description information 1 to the description information 20 belong to the data entry 1 to the data entry 20 respectively. The sequence B1 includes a sequence 1, a sequence 2, ..., and a sequence 20. The sequence 1 to the sequence 20 belong to the data entry 1 to the data entry 20 respectively. The quality score C1 includes a quality score 1, a quality score 2, ..., and a quality score 20. The quality score 1 to the quality score 20 belong to the data entry 1 to the data entry 20 respectively.

It should be noted that, to enable the first file to be accurately restored from the compressed file, in the method 100, in addition to S102, length information of each data entry in each block further needs to be recorded. Because description information in a data entry can be accurately restored according to a feature of the data entry, length information of the data entry may be a length of a sequence or a quality score in the data entry, and lengths of a sequence and a quality score in a same data entry are consistent. In this way, the block 1 may be represented as a block 1 { {a length information set 1}, the description information A1, the sequence B1, the quality score C1}. The length information set 1 may include a length 1 of the sequence 1, a length 2 of the sequence 2, ..., and a length 20 of the sequence 20.

S103: Obtain, based on the file header and the plurality of blocks, a data block corresponding to each of the plurality of blocks.

An implementation of S103 may include: performing adaptive compression on each of the plurality of blocks based on compression parameters in the file header that correspond to different data types, and placing a compression result into a corresponding data block. In an example, S103 may include the following S1031 to S1033. An execution sequence of S1031, S1032, and S1033 is not limited in this embodiment of this application, and S1031, S1032, and S1033 may be performed in any sequence, or may be performed simultaneously.

S1031: Compress the first description information in each of the plurality of blocks based on the description information compression parameter, to obtain a description information compression result corresponding to each of the plurality of blocks.

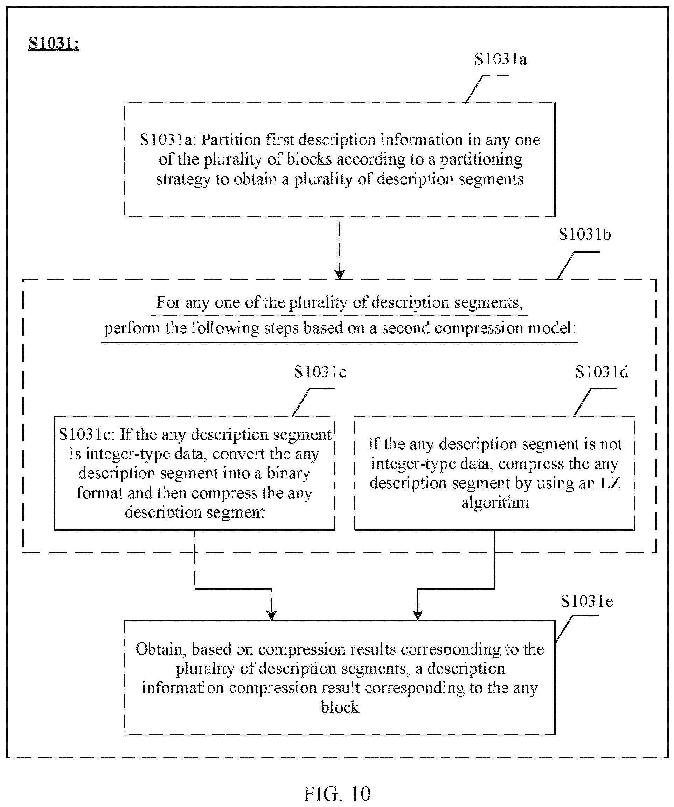

In an example, if the description information compression parameter in the file header includes a partitioning strategy and a second compression model, as shown in FIG. 10, for example, S1031 may include the following steps: S1031a: Partition first description information in any one of the plurality of blocks according to the partitioning strategy to obtain a plurality of description segments. S1031b: For any one of the plurality of description segments, perform the following steps based on the second compression model: S1031c: If the any description segment is integer-type data, convert the any description segment into a binary format and then compress the any description segment. S1031d: If the any description segment is not integer-type data, compress the any description segment by using an LZ algorithm. S1031e: Obtain, based on compression results corresponding to the plurality of description segments, the description information compression result corresponding to the any block. The partitioning strategy may include indication information that is in the description information compression parameter and that indicates whether semantic word segmentation is to be performed. If it is determined, based on the description information compression parameter, that semantic word segmentation needs to be performed, semantic word segmentation is performed on description information during partitioning in S1031a. Alternatively, if the description information compression parameter indicates that semantic word segmentation does not need to be performed, or the description information compression parameter does not include the indication information indicating whether semantic word segmentation is to be performed, a special symbol (for example, a comma, a semicolon, or a colon) in description information may be used as the partitioning strategy in S1031a to perform partitioning. Alternatively, if the description information compression parameter indicates that semantic word segmentation needs to be performed, multi-partitioning may be performed on description information based on a special symbol and semantics.

It should be noted that the second compression model in the description information compression parameter may be the compression process in S1031c to S1031e. The second compression model may include a plurality of compression algorithms (which may also be referred to as encoding algorithms), for example, may include the LZ algorithm in the example and an algorithm corresponding to S1031c.

S1032: Compress the first sequence in each of the plurality of blocks based on the sequence compression parameter, to obtain a sequence compression result corresponding to each of the plurality of blocks.

In an example, if the sequence compression parameter in the file header includes a packing length and a third compression model, S1032 may include, for example, the following steps: S1032a: Process, according to an N-pack strategy, a first sequence in any one of the plurality of blocks to obtain a fourth sequence, where N is an integer greater than 1, and N matches the packing length. S1032b: Compress the fourth sequence based on the third compression model to obtain the sequence compression result corresponding to the any block.

It can be learned that, in this embodiment of this application, during compression of a sequence in each block, an LZ fast matching algorithm is used. Compared with a current LZ algorithm, proposed improvements include: First, N adjacent elements in a raw sequence (for example, bases in a base sequence) form a group according to the N-pack strategy to obtain a new symbol, and then these new symbols are compressed to form a new sequence. This corresponds to S1032a. Second, a matching strategy for the sequence is adjusted according to a characteristic of the sequence based on a general-purpose open-source Zstandard algorithm, to skip matching of a short sequence and improve compression efficiency. This corresponds to S1032b.

It is assumed that the first sequence includes ACCCTCGCAATC, and N=3. Therefore, ACC corresponds to a1, CTC corresponds to a2, GCA corresponds to a3, and ATC corresponds to a4. In this case, the fourth sequence sequentially includes a1, a2, a3, and a4. If the fourth sequence includes a1, a2, a6, a4, ..., a3, a2, a6, a5, ..., a length of a short sequence for which matching is to be skipped needs to be less than three characters. Two characters in the foregoing sequence are repeated. This meets the condition for skipping matching of a short sequence. Therefore, it is not considered that the two portions of bold-faced a2 and a6 match. If the condition for skipping matching of a short sequence is not met, only one of a plurality of matching strings needs to be recorded. For another string matching the recorded string, only information like a positional relationship or a distance between the another string and the recorded string is recorded.

S1033: Compress the first quality score in each of the plurality of blocks based on the quality score compression parameter, to obtain a quality score compression result corresponding to each of the plurality of blocks.

The quality score compression parameter may include at least a first parameter and a second parameter. The first parameter indicates a preceding-context model. The preceding-context model is used to determine a feature value of a preceding-context string of a to-be-encoded character. For related descriptions of the first parameter, refer to the descriptions of the modelID in FIG. 3. The second parameter is used to determine a compression model corresponding to the feature value. For related descriptions of the second parameter, refer to the descriptions of the Compressors in FIG. 3.

In an example, S1033 may include, for example, the following steps: S1033a: For any one of a plurality of quality score characters in any one of the plurality of blocks, obtain a preceding-context string of the any quality score character. S1033b: Determine a feature value of the any quality score character based on the first parameter and the preceding-context string of the any quality score character. S1033c: Determine a target compression model based on the second parameter and the feature value of the any quality score character. S1033d: Compress the any quality score character based on the target compression model. S1033e: Obtain, based on compression results of the plurality of quality score characters, a compression result of the first quality score corresponding to the any block. The feature value may also be referred to as a conditional parameter.

For example, a sequence sequentially includes characters a0 to a14, and the preceding-context model corresponds to preceding context of four characters. Corresponding feature values calculated through S1033a and S1033b may be shown in Table 1.

Table 1

| Sequence | a0 | a1 | a2 | a3 | a4 | a5 | a6 | a7 | a8 | a9 | a10 | a11 | a12 | a13 | a14 |
|---|---|---|---|---|---|---|---|---|---|---|---|---|---|---|---|
| Feature value | 1 | 2 | 3 | 3 | 1 | 2 | 1 | 3 | 2 | 2 | 3 | 1 | 1 | 3 | 2 |

Implementations of S1033a and S1033b may be understood based on this example. For example, calculation of a feature value of a4 is used as an example. It can be determined that a 4-character preceding-context string of a4 is a0 to a3. It is assumed that lengths of a0 to a3 are 1 bit, 1 bit, 2 bits, and 4 bits respectively after compression. For example, the feature value corresponding to a4 may be calculated by using the following formula (1):

$$F(a0,a1,a2,a3) = T1(a3) + (D_2(a2,a3) << 4) + (D_2(a1,a2) << 6) + (D_1(a0,a1) << 7) \quad \text{Formula (1)}$$

F(a0, a1, a2, a3) is the feature value of a4. T1() may be implemented by using an empirical table, an empirical correspondence, or a preset function relationship in the preceding-context model. The empirical table is used as an example. T1 may be represented as $[0, ..., 255] \rightarrow [0, ..., 16]$. T1(a3) may be a value, ranging from 0 to 16, to which a3 that ranges from 0 to 255 is mapped. $D_2(y, x)$ is a function for storing a case of a difference. For example, when x=y, $D_2(y, x)=0$; when x<y, $D_2(y, x)=1$; or when x>y, $D_2(y, x)=2$. $D_1(y, x)$ is a function that does not store a case of whether values are the same. For example, when x=y, $D_1(y, x)=0$; or when x and y are not equal, $D_1(y, x)=1$. "<<" is a left-shift symbol. An object to be left-shifted is before "<<". A quantity of bits by which a left shift is to be performed is after "<<". The quantity of bits by which the left shift is to be performed is determined based on an order of preceding-context characters in the preceding-context string and bits obtained after the preceding-context characters are compressed.

For a3 that is adjacent to a4, to indicate that a3 has greatest impact on an occurrence of a4, impact of a3 on a feature value of a4 is indicated by an empirical table, an empirical correspondence, or a preset function relationship. For a1 and a2 that are close to a4, impact of a1 and a2 on the feature value of a4 may be indicated by $D_2(y, x)$ that can indicate a large amount of information. For a0 that is farthest away, impact of a0 on the feature value of a4 may be indicated by $D_1(y, x)$ that can indicate a small amount of information.

It should be noted that a feature value of a character without a preceding-context string meeting a preset length (for example, 4 bytes) may be implemented based on a preceding-context string with a smaller length in another calculation manner, or may be obtained based on experience or in another manner. This is not limited in this embodiment of this application.

After a feature value of each quality score character is calculated, a target compression model (which may also be referred to as an encoding model or a probabilistic model) applicable to each quality score character may be determined based on the feature value and the second parameter, and each quality score character is compressed by using a corresponding target compression model, to obtain a compression result of each quality score character. In this way, the compression result of the first quality score is obtained. This process is a dynamic encoding scheme.

It should be noted that, during execution of S1033a to S1033e, S1033a and S1033b may be first performed to calculate feature values of all quality score characters in the first quality score in the block. Then, in one case, S1033c and S1033d are performed on each quality score character bit by bit according to an order in which the quality score characters appear in the first quality score, to obtain a compression result of each quality score character. In this process, a historically used target compression model needs to be continuously maintained, and a historical feature value corresponding to a previous same feature value needs to be updated by using a most recently used target compression model, causing high pressure on a memory and processing. In another case, S1033c and S1033d may alternatively be performed simultaneously on a plurality of quality score characters with a same feature value based on feature values of the quality score characters, to obtain compression results of the plurality of quality score characters corresponding to the feature value. In this way, quality score characters are classified based on feature values, to reduce a quantity of times that S1033c and S1033d are performed. In addition, after S1033c and S1033d are performed on each feature value, a target compression model corresponding to the feature value may not be maintained in the memory, so that fast and efficient "static" encoding can be implemented, and pressure on the memory and processing is also low.

In some implementations, S1033c may include: sorting quality score characters in the any block based on the feature value to obtain a plurality of quality score strings, where each of the plurality of quality score strings corresponds to a same feature value; and for a first quality score string among the plurality of quality score strings, determining a first compression model based on a feature value corresponding to the first quality score string. In this case, S1033d may include: compressing the first quality score string based on the first compression model.

Table 1 is used as an example. Three quality score strings may be obtained after S1033c is performed. For details, refer to Table 2.

Table 2

| Sequence | a0 | a4 | a6 | a11 | a12 | a1 | a5 | a8 | a9 | a14 | a2 | a3 | a7 | a10 | a13 |
|---|---|---|---|---|---|---|---|---|---|---|---|---|---|---|---|
| Feature value | 1 | 1 | 1 | 1 | 1 | 2 | 2 | 2 | 2 | 2 | 3 | 3 | 3 | 3 | 3 |

The first quality score string may be a0, a4, a6, a11, and a12, and the feature value corresponding to the first quality score string is 1. Alternatively, the first quality score string may be a1, a5, a8, a9, and a14, and the feature value corresponding to the first quality score string is 2. Alternatively, the first quality score string may be a2, a3, a7, a10, and a13, and the feature value corresponding to the first quality score string is 3. It should be noted that a specific process of grouping quality score characters may include the following steps: Step 1: Count a quantity of occurrences of each feature value as 5 and a quantity of different feature values as 3. Step 2: Set pointers whose quantity is the same as a quantity, that is, 3, of occurrences of different feature values, where positions indicated by the pointers are determined based on the quantity of occurrences of the features. For example, a pointer 1 points to a position of the 0th character, a pointer 2 points to a position of the 5th character, and a pointer 3 points to a position of the 10th character. Step 3: For each character in Table 1, sequentially insert, based on a feature value of the character, the character in a position to which a pointer corresponding to the feature value points, where when the character is inserted, the pointer points to a next character position.

For example, the first quality score string is a0, a4, a6, a11, and a12. S1033d may be specifically: determining, based on a feature value 1 and the second parameter, a first compression model corresponding to 1, and inputting the string a0, a4, a6, a11, and a12 to the first compression model, so that the first compression model outputs a compression result of the string.

It is assumed that the compression result of the string a0, a4, a6, a11, and a12 is a compression result 1, a compression result of the string a1, a5, a8, a9, and a14 is a compression result 2, and a compression result of the string a2, a3, a7, a10, and a13 is a compression result 3. In this case, the compression result of the first quality score in the block includes the compression result 1, the compression result 2, and the compression result 3.

It should be noted that, in a solution in which quality score characters are compressed after being reordered, to ensure effective decompression, a plurality of reordered quality score strings need to be restored to an original order, and a feature value of the 1st quality score character may be further recorded in a block. For example, in the examples shown in Table 1 and Table 2, a feature value of a0 may be recorded as 1. In this case, after the compression result of the first quality score is decompressed, the sequence shown in Table 2 is obtained. Then a feature value of a1 is calculated based on the feature value of a0. If the feature value of a1 is 1, it is considered that a0 is followed by a1, and a position of a1 remains unchanged. If the feature value of a1 is 2, the 1st character in a quality score string with a feature value of 2 is extracted to a character position after a0. If the feature value of a1 is 3, the 1st character in a quality score string with a feature value of 3 is extracted to a character position after a0. By analogy, after a feature value of each character is calculated, a corresponding character is extracted from a corresponding quality score string based on the feature value, until all quality score characters are restored to original positions.

In some implementations, in the solution in which quality score characters are compressed after being reordered, to further improve compression efficiency, the quality score compression parameter in the file header may further include a third parameter. The third parameter indicates a threshold of a string length for performing a compression operation. For related descriptions of the third parameter, refer to the descriptions of the cutSize in FIG. 3. In this implementation, for each of the plurality of quality score strings, whether a length of the quality score string is less than the threshold of a string length is first determined. If no, it is considered that the length of the quality score string meets a compression standard, and S1033c and S1033d are performed on the quality score string. If yes, the quality score string is temporarily not compressed, and is stored in a target area. In this way, to-be-compressed quality score characters are accumulated in the target area. When a length of a quality score string in the target area reaches a preset length (which may be the same as or different from the threshold of a string length that is indicated by the third parameter). Alternatively, when the foregoing determining is completed for all quality score strings in the block, a quality score string in the target area is compressed by using a preset general-purpose compression algorithm for compressing data in the target area.

It should be noted that, in this embodiment of this application, a length of a character is counted in bytes. To be specific, one character occupies a space of 8 bits.

It can be learned that a lightweight FASTQ compression algorithm of optimal binning and fast entropy coding is performed on the first quality score in each block. A compression ratio reaches a SOTA level. No reference needs to be made to an input of a sequence, so that a small memory is occupied, and compression efficiency is significantly improved. Compared with a complex model in commonly used gzip or gtz, this compression algorithm has a significant advantage. When data is partitioned by 2 megabytes (Megabytes, MB), gene data exceeding 700 gigabytes (Gigabyte, GB) is separately tested by using gzip, gtz, and the FASTQ compression algorithm. For specific comparison, refer to Table 3.

Table 3

| Compression algorithm | gzip | gtz | **FASTQ compression algorithm** |
|---|---|---|---|
| Compression ratio | 3.09 | 5.3 | **5.4** |
| Compression/Decompression throughput (Mbps/core) | 20/100 | 15/15 | **70/160** |

The Mbps/core may indicate that million bits are transmitted per second per core, and the Mbps stands for million bits per second.

S1031 to S1033 are performed on each block to obtain a description information compression result, a sequence compression result, and a quality score compression result of the block. In addition, recorded length information is compressed to obtain a length information compression result. Finally, various compression results are stored, according to the file format defined in embodiments of this application, in a data block corresponding to the block. For details, refer to related descriptions of FIG. 4 and FIG. 5.

S104: Obtain a second file, where the second file is a file obtained by compressing the first file, and the second file includes the file header and the data block corresponding to each of the plurality of blocks.

After a plurality of data blocks are obtained, the plurality of data blocks are sequentially arranged between the file header and the ending block to obtain the second file.

The description information compression result may include at least a size of compressed description information and a first compressed stream corresponding to description information, where the first compressed stream includes data obtained by compressing the description information. The sequence compression result may include at least a size of a compressed sequence and a second compressed stream corresponding to a sequence, where the second compressed stream includes data obtained by compressing the sequence. The quality score compression result may include at least a size of a compressed quality score and a third compressed stream corresponding to a quality score, where the third compressed stream includes data obtained by compressing the quality score.

To facilitate random reading from a FASTQ file and implement an operation on an ultra-large file, some optional blocks may be extended, as a directory area, between a file header and the $1^{st}$ data block or between the last data block and an ending block. For example, the directory area may be shown in FIG. 8. The directory area includes directory blocks whose quantity is the same as a quantity of data blocks. For a format of each target block, refer to the format of the optional block shown in FIG. 7. For a directory block included in the second file, the target block may indicate a sequence number of the $1^{st}$ data entry in a block, a position of the block in the second file, and a position of the block in the first file.

It should be noted that, after the second file is obtained, when the second file needs to be decompressed, the method 100 may further include: decompressing, based on the file header of the second file, the plurality of data blocks in the second file to obtain the first file.

During specific implementation, a decompression process may include the following steps: Step 1: Obtain the file header of the second file. Step 2: Correspondingly decompress compressed data in each data block based on a parameter in the file header. Step 3: Obtain a third file based on a decompression result of each data block. If the second file is secure and the decompression process is correct, the third file is the same as the first file. In step 2, a process of decompressing compressed data in a specific data block may include: correspondingly decompressing a length information compression result, a description information compression result, a sequence compression result, and a quality score compression result separately; and restoring a decompression result of each part of content to a data entry set in a FASTQ file based on a feature of a format of the FASTQ file and content obtained by decompressing the length information compression result. In step 3, data entry sets obtained by decompressing data blocks may be combined into the third file.

It can be learned that, according to the method 100, lightweight compression and decompression of a FASTQ file can be effectively implemented. This not only makes storage of a file with a large amount of data become friendly, but also greatly improves compression efficiency of the file with a large amount data.

The foregoing method for compressing and decompressing a quality score in a FASTQ file can adapt to other data that can be compressed according to the foregoing descriptions.

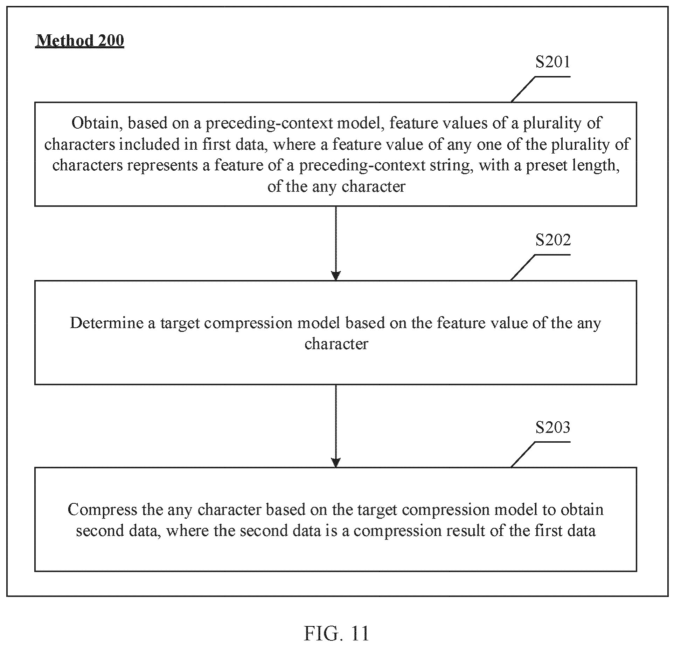

As shown in FIG. 11, an embodiment of this application further provides a data processing method 200. For example, the method 200 may include the following S201 to S203.

S201: Obtain, based on a preceding-context model, feature values of a plurality of characters included in first data, where a feature value of any one of the plurality of characters represents a feature of a preceding-context string, with a preset length, of the any character.

In an example, S201 may include: for any one of the plurality of characters, obtaining a preceding-context string, with a preset length, of the any character; and determining a feature value of the any character based on a length of each compressed preceding-context character in the preceding-context string, each preceding-context character in the preceding-context string, and a preset calculation rule. For example, for the preset calculation rule, refer to the foregoing formula (1) and related descriptions.

It should be noted that, for an implementation and achieved technical effect of S201, reference may be made to related descriptions in S1033b.

The first data may be a first quality score in any block obtained by performing S101 on a FASTQ file.

S202: Determine a target compression model based on the feature value of the any character.

S203: Compress the any character based on the target compression model to obtain second data, where the second data is a compression result of the first data.

In an example, S202 and S203 may include: for each character, determining, based on a feature value of the character, a target compression model applicable to the character, and then compressing the character by using the target compression model. In this way, dynamic compression is performed per character. Because feature values of all characters have been centrally pre-calculated in S201, although a historically used target compression model needs to be continuously maintained and a historical feature value corresponding to a previous same feature value needs to be updated by using a most recently used target compression model, causing high pressure on a memory and processing, efficiency is still improved compared with a current compression scheme.

In another example, S202 may include: sorting a plurality of characters in the first data based on the feature value to obtain a plurality of strings, where each of the plurality of strings corresponds to a same feature value; and for the $1^{st}$ one of the plurality of strings, determining a first compression model based on a mapping relationship and a feature value

corresponding to the 1st string, where the mapping relationship includes a correspondence between a plurality of groups of feature values and compression models. In this case, for example, S203 may include: compressing the 1st string based on the first compression model to obtain third data, where the second data includes the third data. In this way, characters are classified based on feature values, to reduce a quantity of times that S202 and S203 are performed. In addition, after S202 and S203 are performed on each feature value, a target compression model corresponding to the feature value may not be maintained in a memory, so that fast and efficient "static" encoding can be implemented, and pressure on the memory and processing is also low.

It should be noted that, for implementations and achieved technical effect of S202 and S203, reference may be made to related descriptions in S1033c to S1033e.

In the method 200, the preceding-context model may be understood as a higher-order prediction model with an 8-bit parameter space. When the first data is a character sequence a1, a2, ..., an, ..., a $k^{th}$-order preceding-context model attempts to preset a probability distribution of a current character by using the first k characters at a current position. In this embodiment of this application, k=4 is used as an example for related description. To be specific, for any character an (n>4), a probability that each character may occur at a position n is predicted by using values of four symbols: a(n-4), a(n-3), a(n-2), and a(n-1). An output of the prediction is a distribution Pn, and Pn provides a probability that each possible symbol occurs at the position n. If one byte is considered as one character, a value of the character ranges from 0 to 255, and an information amount is 8 bits. If there are four characters, an information amount is 32 bits. Each possible four-character preceding context of a fourth-order model is used as a parameter space, and a size of the parameter space is 32 bits. This value is quite large because the space size is doubled each time a length of the preceding context is increased by 1 bit. However, the preceding-context model provided in this embodiment of this application can be an efficient preceding-context model with a 32-bit space. If fqzcomp is used, 16 bits need to be occupied. However, if the preceding-context model provided in this embodiment of this application is used, a size can be further compressed to 8 bits, which is 256 times smaller than that of a model in fqzcomp, but prediction effect almost remains unchanged. A mature higher-order encoder can be quite easily concatenated to the end of the 8-bit model. Therefore, the 8-bit solution is not only more advanced than the 16-bit technology, but also more practical.

In addition, in the method 200, a compression scheme of a higher-order model with dynamic encoding can be further replaced with static encoding. If a character a needs to be encoded through entropy coding, a predicted probability p(a) of the character a needs to be known. If code text encoded by using an entropy coding algorithm needs to be decoded, predicted probabilities (namely, an overall distribution) of all possible characters need to be known. Therefore, in the entropy coding algorithm, a distribution of an entire probabilistic model (namely, a compression model) needs to be maintained during encoding or decoding. For example, when a character is 1 byte, an entire string is all possible byte values, that is, 0 to 255. To use the entropy coding algorithm, predicted probabilities of 256 possible values need to be maintained in the memory at all times. When the preceding-context model is a higher-order conditional probabilistic model, each character has more than one conditional probability, and therefore there are a plurality of conditional probabilities. Currently, all probability values are stored in the memory. For each character, in a string, that needs to be encoded, a corresponding probability value is read and selected based on a condition (for example, a preceding-context value) to which the character belongs. Each conditional probability distribution may be considered as a submodel (because the submodel records a complete valid distribution). For example, 16-bit byte-level prediction is used in fqzcomp. Therefore, there are 256 characters, each character has 2^16=65536 possible submodels, a total of 256×65536=16777216 bits need to be stored, and there are approximately 16 MB probabilities. If one probability is represented by one 32-bit (namely, 4-byte) floating-point number, a memory of 64 MB is needed. If prediction is performed at a bit level, in the foregoing example, because a meaning of a quality score is provided by one complete byte and a most significant bit and a least significant bit also need to be used as conditions, a model size does not change. In this application, 8-bit byte-level prediction is used, and only 65536 (namely, 64 KB) possible probability values need to be stored. However, in a current CPU, at this size, even if only a short floating-point number is used, a memory of 128 KB is still occupied. In addition, in the compression model, not all parameters can be placed into a level-1 cache. During encoding, due to a random change in a preceding context, a data structure of the compression model needs to be randomly read and updated, leading to degradation of performance to some extent. In this embodiment of this application, characters are simply reordered to arrange together all characters whose preceding contexts have a same feature, and a size of the subsequence is recorded. Therefore, there is no requirement for random reading from a large data structure, and static encoding may be introduced to implement compression, so that encoding efficiency is improved. In addition, in the method provided in this embodiment of this application, because not all compression models need to be stored or maintained, the method can operate with a quite small memory requirement.

It should be noted that, in the technical solution provided in this embodiment of this application, a defined high-throughput and high-compression-ratio data format of a FASTQ file and a designed data processing (namely, data compression) process may be configured and used. In a scenario similar to compression of a FASTQ file, efficient lightweight compression and storage can be implemented.

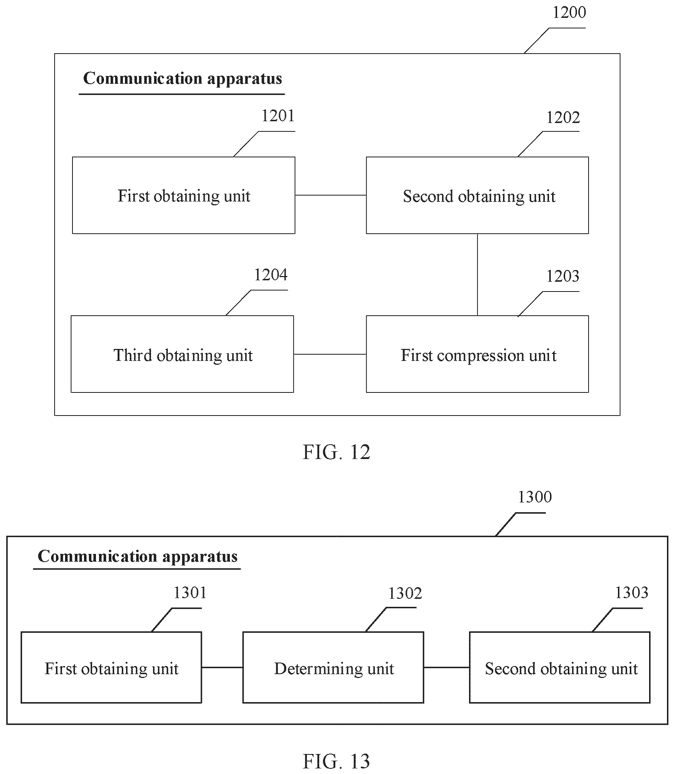

Correspondingly, an embodiment of this application further provides a communication apparatus 1200, as shown in FIG. 12. The communication apparatus 1200 may include a first obtaining unit 1201, a second obtaining unit 1202, a first compression unit 1203, and a third obtaining unit 1204.

The first obtaining unit 1201 is configured to obtain a file header, where the file header includes block information, a description information compression parameter, a sequence compression parameter, and a quality score compression parameter. The first obtaining unit 1201 may perform S101 shown in FIG. 9.

The second obtaining unit 1202 is configured to obtain a plurality of blocks based on the block information and a first file, where each of the plurality of blocks includes first description information, a first sequence, and a first quality score. The second obtaining unit 1202 may perform S 102 shown in FIG. 9.

The first compression unit 1203 is configured to compress the first description information, the first sequence, and the first quality score in each of the plurality of blocks based on the description information compression parameter, the sequence compression parameter, and the quality score compression parameter to obtain a data block corresponding to each of the plurality of blocks, where the data block includes a description information compression result, a sequence compression result, and a quality score compression result of a corresponding block. The first compression unit 1203 may perform S103 shown in FIG. 9.

The third obtaining unit 1204 is configured to obtain a second file, where the second file is a file obtained by compressing the first file, and the second file includes the file header and the data block corresponding to each of the plurality of blocks. The third obtaining unit 1204 may perform S 104 shown in FIG. 9.

Optionally, the second obtaining unit 1202 may include a block division subunit and a first obtaining subunit. The block division subunit is configured to perform block division on the first file based on the block information to obtain a plurality of data entry sets, where each of the plurality of data entry sets includes at least one data entry. The first obtaining subunit is configured to classify a data entry in any one of the plurality of data entry sets based on a data type to obtain a block corresponding to the any data entry set, where the data type includes at least one of description information, a sequence, and a quality score.

Optionally, the block information includes a memory size identifier memID, and the block division subunit is specifically configured to: determine memory information based on the memID; and perform block division on the first file based on the memory information to obtain the plurality of data entry sets.

Optionally, the any data entry set includes a first data entry and a second data entry, the first data entry includes second description information, a second sequence, and a second quality score, and the second data entry includes third description information, a third sequence, and a third quality score. Therefore, in the block corresponding to the any data entry set, the first description information includes the second description information and the third description information, the first sequence includes the second sequence and the third sequence, and the first quality score includes the second quality score and the third quality score.

Optionally, the block corresponding to the any data entry set further includes length information of each data entry in the any data entry set, the length information is a length of a sequence or a quality score in a corresponding data entry, and the data block further includes data obtained by compressing length information of each data entry in the corresponding block.

Optionally, the quality score compression parameter includes a first parameter and a second parameter, the first parameter indicates a preceding-context model, the preceding-context model is used to determine a feature value of a preceding-context string of a to-be-encoded character, and the second parameter is used to determine a compression model corresponding to the feature value.

Optionally, the first compression unit 1203 may include a second obtaining subunit, a first determining subunit, a second determining subunit, a first compression subunit, and a third obtaining subunit. The second obtaining subunit is configured to: for any one of a plurality of quality score characters in any one of the plurality of blocks, obtain a preceding-context string of the any quality score character. The first determining subunit is configured to determine a feature value of the any quality score character based on the first parameter and the preceding-context string of the any quality score character. The second determining subunit is configured to determine a target compression model based on the second parameter and the feature value of the any quality score character. The first compression subunit is configured to compress the any quality score character based on the target compression model. The third obtaining subunit is configured to obtain, based on compression results of the plurality of quality score characters, a compression result of the first quality score corresponding to the any block.

Optionally, the second determining subunit is specifically configured to: sort quality score characters in the any block based on the feature value to obtain a plurality of quality score strings, where each of the plurality of quality score strings corresponds to a same feature value; and for a first quality score string among the plurality of quality score strings, determine a first compression model based on a feature value corresponding to the first quality score string. In this case, the first compression subunit is specifically configured to compress the first quality score string based on the first compression model.

Optionally, the second file further includes a feature value of the 1st quality score character in the any block.

Optionally, the quality score compression parameter further includes a third parameter, the third parameter indicates a threshold of a string length for performing a compression operation, and the apparatus 1200 may further include a storage unit and a second compression unit. The storage unit is configured to store, in a target area, a quality score string with a length less than the threshold of a string length among the plurality of quality score strings. The second compression unit is configured to compress the quality score string in the target area based on a general-purpose compression algorithm.

Optionally, the description information compression parameter includes a partitioning strategy, a second compression model, and a fourth obtaining subunit, and the first compression unit 1203 may include a partitioning subunit and a second compression subunit. The partitioning subunit is configured to partition first description information in any one of the plurality of blocks according to the partitioning strategy to obtain a plurality of description segments. The second compressing subunit is configured to: for any one of the plurality of description segments, perform the following steps based on the second compression model: if the any description segment is integer-type data, converting the any description segment into a binary format and then compressing the any description segment; or if the any description segment is not integer-type data, compressing the any description segment by using an LZ algorithm. The fourth obtaining subunit is configured to obtain, based on compression results corresponding to the plurality of description segments, the description information compression result corresponding to the any block.

Optionally, the sequence compression parameter includes a packing length and a third compression model, and the first compression unit 1203 may include a processing subunit and a third compression subunit. The processing subunit is configured to process, according to an N-pack strategy, a first sequence in any one of the plurality of blocks to obtain a fourth sequence, where N is an integer greater than 1, and N matches the packing length. The third compression subunit is configured to compress the fourth sequence based on the third compression model to obtain the sequence compression result corresponding to the any block.

Optionally, the description information compression result includes a size of compressed description information and a first compressed stream corresponding to description information, where the first compressed stream includes data obtained by compressing the description information; the sequence compression result includes a size of a compressed sequence and a second compressed stream corresponding to a sequence, where the second compressed stream includes data obtained by compressing the sequence; and the quality score compression result includes a size of a compressed quality score and a third compressed stream corresponding to a quality score, where the third compressed stream includes data obtained by compressing the quality score.

Optionally, the file header further includes at least one of the following fields: a format signature, a file size, a third-line status, or a check bit.

Optionally, the second file further includes a directory block, and the target block indicates a sequence number of the 1st data entry in a block, a position of the block in the second file, and a position of the block in the first file.

Optionally, the first file is a FASTQ file.

Optionally, the apparatus 1200 further includes a decompression unit. The decompression unit is configured to decompress, based on the file header of the second file, a plurality of data blocks in the second file to obtain the first file.

It should be noted that, for various specific embodiments of the communication apparatus 1200, reference may be made to related descriptions of the method 100 corresponding to FIG. 9. Details are not described in this embodiment again.

Correspondingly, an embodiment of this application further provides a communication apparatus 1300, as shown in FIG. 13. The communication apparatus 1300 may include a first obtaining unit 1301, a determining unit 1302, and a second obtaining unit 1303.

The first obtaining unit 1301 is configured to obtain, based on a preceding-context model, feature values of a plurality of characters included in first data, where a feature value of any one of the plurality of characters represents a feature of a preceding-context string, with a preset length, of the any character. The first obtaining unit 1301 may perform S201 shown in FIG. 11.

The determining unit 1302 is configured to determine a target compression model based on the feature value of the any character. The determining unit 1302 may perform S202 shown in FIG. 11.

The second obtaining unit 1303 is configured to compress the any character based on the target compression model to obtain second data, where the second data is a compression result of the first data. The second obtaining unit 1303 may perform S203 shown in FIG. 11.

Optionally, the first obtaining unit 1301 may include a first obtaining subunit and a first determining subunit. The first obtaining subunit is configured to: for any one of the plurality of characters, obtain a preceding-context string, with a preset length, of the any character. The first determining subunit is configured to determine a feature value of the any character based on a length of each compressed preceding-context character in the preceding-context string, each preceding-context character in the preceding-context string, and a preset calculation rule.

Optionally, the determining unit 1302 may include a second obtaining subunit and a second determining subunit. The second obtaining subunit is configured to sort a plurality of characters in the first data based on the feature value to obtain a plurality of strings, where each of the plurality of strings corresponds to a same feature value. The second determining subunit is configured to: for the 1st one of the plurality of strings, determine a first compression model based on a mapping relationship and a feature value corresponding to the 1st string, where the mapping relationship includes a correspondence between a plurality of groups of feature values and compression models. The second obtaining unit 1303 is specifically configured to compress the 1st string based on the first compression model to obtain third data, where the second data includes the third data.

Optionally, the first data is a quality score in any block obtained by dividing a FASTQ file into blocks.

It should be noted that, for various specific embodiments of the communication apparatus 1300, reference may be made to related descriptions of the method 200 corresponding to FIG. 11. Details are not described in this embodiment again.

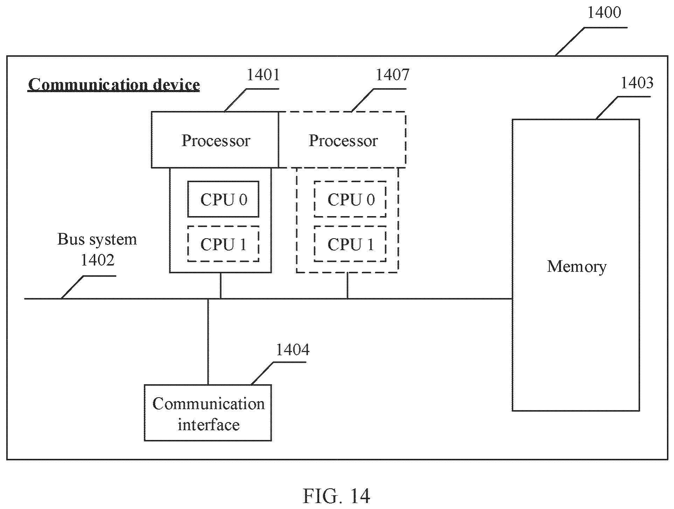

With reference to FIG. 14, an embodiment of this application provides a communication device 1400. The communication device 1400 may be an execution entity in any one of the foregoing embodiments. The communication device 1400 may implement the functions in the foregoing embodiments. The communication device 1400 includes at least one processor 1401, a bus system 1402, a memory 1403, and at least one communication interface 1404.

The communication device 1400 is an apparatus of a hardware structure, and may be configured to implement the functional modules in the communication apparatus 1200 shown in FIG. 12. For example, a person skilled in the art may figure out that the first obtaining unit 1201, the second obtaining unit 1202, the first compression unit 1203, and the third obtaining unit 1204 in the communication apparatus 1200 shown in FIG. 12 may be implemented by the at least one processor 1401 by invoking code in the memory 1403. For another example, a person skilled in the art may figure out that the first obtaining unit 1301, the determining unit 1302, and the second obtaining unit 1303 in the communication apparatus 1300 shown in FIG. 13 may be implemented by the at least one processor 1401 by invoking code in the memory 1403.

Optionally, the communication device 1400 may be further configured to implement a function of a network device or a control entity in any one of the foregoing embodiments.

Optionally, the processor 1401 may be a general-purpose central processing unit (central processing unit, CPU), a network processor (network processor, NP), a microprocessor, an application-specific integrated circuit (application-specific integrated circuit, ASIC), or one or more integrated circuits for controlling program execution for the solutions in this application.

The bus system 1402 may include a channel for transmitting information between the foregoing components.

The communication interface 1404 is configured to communicate with another device or a communication network.

The memory 1403 may be a read-only memory (read-only memory, ROM) or another type of static storage device that can store static information and instructions, or a random access memory (random access memory, RAM) or another type of dynamic storage device that can store information and instructions; or may be an electrically erasable programmable read-only memory (electrically erasable programmable read-only memory, EEPROM), a compact disc read-only memory (compact disc read-only memory, CD-ROM) or another compact disc storage, an optical disc storage (including a compact disc, a laser disc, an optical disc, a digital versatile disc, a Blu-ray disc, and the like), a magnetic disk storage medium or another magnetic storage device, or any other medium that can carry or store expected program code in a form of instructions or data structures and that is accessible to a computer, but is not limited thereto. The memory may exist independently, and is connected to the processor through a bus. The memory may alternatively be integrated with the processor.

The memory 1403 is configured to store application program code for executing the solutions in this application, and the execution is controlled by the processor 1401. The processor 1401 is configured to execute the application program code stored in the memory 1403, to implement the functions in the method in this application.

During specific implementation, in an embodiment, the processor 1401 may include one or more CPUs, for example, a CPU 0 and a CPU 1 in FIG. 14.

During specific implementation, in an embodiment, the communication device 1400 may include a plurality of processors, for example, the processor 1401 and a processor 1407 in FIG. 14. Each of the processors may be a single-core (single-CPU) processor or a multi-core (multi-CPU) processor. The processor herein may be one or more devices, circuits, and/or processing cores for processing data (for example, computer program instructions).

It should be understood that the communication apparatus and the communication device in the foregoing product forms respectively have any functions implemented by the execution entity in the foregoing method embodiments. Details are not described herein again.

An embodiment of this application further provides a chip, including a processor and an interface circuit. The interface circuit is configured to receive an instruction and transmit the instruction to the processor. The processor may be, for example, a specific implementation form of the packet processing apparatus in embodiments of this application, and may be configured to perform the method 100 or the method 200. The processor is coupled to a memory. The memory is configured to store a program or instructions. When the program or the instructions are executed by the processor, the chip system is enabled to implement the method in any one of the foregoing method embodiments.

Optionally, the chip system may include one or more processors. The processor may be implemented by using hardware or software. When the processor is implemented by using hardware, the processor may be a logic circuit, an integrated circuit, or the like. When the processor is implemented by using software, the processor may be a general-purpose processor, and is implemented by reading software code stored in the memory.

Optionally, the chip system may also include one or more memories. The memory may be integrated with the processor, or may be disposed separately from the processor. This is not limited in this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in this application.

For example, the chip system may be a field programmable gate array (field programmable gate array, FPGA), an application-specific integrated circuit (application specific integrated circuit, ASIC), a system-on-a-chip (system on chip, SoC), a central processing unit (central processing unit, CPU), a network processor (network processor, NP), a digital signal processing circuit (digital signal processor, DSP), a microcontroller (microcontroller unit, MCU), a programmable controller (programmable logic device, PLD), or another integrated chip.

In addition, an embodiment of this application further provides a storage medium. The storage medium stores program code or instructions. When the program code or the instructions are run on a processor, the processor is enabled to perform the method in any one of the implementations in the foregoing embodiments.

In addition, an embodiment of this application further provides a program product. When the program product is run on a processor, the processor is enabled to perform the method in any one of the implementations of the method 100 or the method 200.

It should be understood that "determining B based on A" mentioned in embodiments of this application does not mean that B is determined only based on A, and B may alternatively be determined based on A and/or other information.

It should be understood that a network architecture and a service scenario described in embodiments of this application are intended to describe the technical solutions in embodiments of this application more clearly, and do not constitute a limitation on the technical solutions provided in embodiments of this application. A person of ordinary skill in the art can know that the technical solutions provided in embodiments of this application are also applicable to similar technical problems with evolution of the network architecture and emergence of a new service scenario.

Ordinal numbers such as "1", "2", "3", "first", "second", and "third" in this application are intended to distinguish between a plurality of objects, but not to limit a sequence of the plurality of objects.

"A and/or B" mentioned in this application should be understood as including the following cases: Only A is included, only B is included, or both A and B are included.

From the foregoing descriptions of the implementations, a person skilled in the art may clearly understand that some or all of the steps of the methods in the foregoing embodiments may be implemented by software in combination with a general-purpose hardware platform. Based on such an understanding, the technical solutions in this application may be implemented in a form of a software product. The computer software product may be stored in a storage medium, for example, a read-only memory (English: read-only memory, ROM)/RAM, a magnetic disk, or a compact disc, and includes several instructions for instructing a computer device (which may be a personal computer, a server, or a network communication device like a router) to perform the methods described in embodiments of this application or in some parts of embodiments.

All embodiments in this specification are described in a progressive manner. For same or similar parts in embodiments, refer to the embodiments. Each embodiment focuses on a difference from other embodiments. In particular, the system embodiment and the device embodiment are basically similar to the method embodiments, and therefore are described briefly. For related parts, refer to the descriptions in the method embodiments. The described device embodiment and system embodiment are merely examples. The modules described as separate components may or may not be physically separated, and components shown as modules may or may not be physical modules, to be specific, may be located in one place, or may be distributed on a plurality of network units. Some or all of the modules may be selected according to actual requirements to achieve the objectives of the solutions of embodiments. A person of ordinary skill in the art may understand and implement the solutions without creative efforts.

The foregoing descriptions are merely example implementations of this application, but are not intended to limit the protection scope of this application. It should be noted that a person of ordinary skill in the art may make improvements and polishing without departing from this application, and the improvements and polishing shall fall within the protection scope of this application.

**Claims**

**1.** A data processing method, comprising:

obtaining a file header, wherein the file header comprises block information, a description information compression parameter, a sequence compression parameter, and a quality score compression parameter;

obtaining a plurality of blocks based on the block information and a first file, wherein each of the plurality of blocks comprises first description information, a first sequence, and a first quality score;

compressing the first description information, the first sequence, and the first quality score in each of the plurality of blocks based on the description information compression parameter, the sequence compression parameter, and the quality score compression parameter to obtain a data block corresponding to each of the plurality of blocks, wherein the data block comprises a description information compression result, a sequence compression result, and a quality score compression result of a corresponding block; and

obtaining a second file, wherein the second file is a file obtained by compressing the first file, and the second file comprises the file header and the data block corresponding to each of the plurality of blocks.

2.  The method according to claim 1, wherein obtaining the plurality of blocks based on the block information and the first file comprises:

   performing block division on the first file based on the block information to obtain a plurality of data entry sets, wherein each of the plurality of data entry sets comprises at least one data entry; and

   classifying a data entry in any one of the plurality of data entry sets based on a data type to obtain a block corresponding to the any data entry set, wherein the data type comprises at least one of description information, a sequence, and a quality score.

3.  The method according to claim 2, wherein the block information comprises a memory size identifier memID, and performing block division on the first file based on the block information to obtain the plurality of data entry sets comprises:

   determining memory information based on the memID; and

   performing block division on the first file based on the memory information to obtain the plurality of data entry sets.

4.  The method according to claim 2 or 3, wherein the any data entry set comprises a first data entry and a second data entry, the first data entry comprises second description information, a second sequence, and a second quality score, and the second data entry comprises third description information, a third sequence, and a third quality score, and therefore in the block corresponding to the any data entry set, the first description information comprises the second description information and the third description information, the first sequence comprises the second sequence and the third sequence, and the first quality score comprises the second quality score and the third quality score.

5.  The method according to any one of claims 2 to 4, wherein the block corresponding to the any data entry set further comprises length information of each data entry in the any data entry set, the length information is a length of a sequence or a quality score in a corresponding data entry, and the data block further comprises data obtained by compressing length information of each data entry in the corresponding block.

6.  The method according to any one of claims 1 to 5, wherein the quality score compression parameter comprises a first parameter and a second parameter, the first parameter indicates a preceding-context model, the preceding-context model is used to determine a feature value of a preceding-context string of a to-be-encoded character, and the second parameter is used to determine a compression model corresponding to the feature value.

7.  The method according to claim 6, wherein compressing the first quality score in each of the plurality of blocks based on the quality score compression parameter comprises:

   for any one of a plurality of quality score characters in any one of the plurality of blocks, obtaining a preceding-context string of the any quality score character;

   determining a feature value of the any quality score character based on the first parameter and the preceding-context string of the any quality score character;

   determining a target compression model based on the second parameter and the feature value of the any quality score character;

   compressing the any quality score character based on the target compression model; and

   obtaining, based on compression results of the plurality of quality score characters, a compression result of the first quality score corresponding to the any block.

8.  The method according to claim 7, wherein

determining the target compression model based on the second parameter and the feature value of the any quality score character comprises:

> sorting quality score characters in the any block based on the feature value to obtain a plurality of quality score strings, wherein each of the plurality of quality score strings corresponds to a same feature value; and
> for a first quality score string among the plurality of quality score strings, determining a first compression model based on a feature value corresponding to the first quality score string; and
> compressing the any quality score character based on the target compression model comprises:
> compressing the first quality score string based on the first compression model.

9. The method according to claim 8, wherein the second file further comprises a feature value of the $1^{st}$ quality score character in the any block.

10. The method according to claim 8 or 9, wherein the quality score compression parameter further comprises a third parameter, the third parameter indicates a threshold of a string length for performing a compression operation, and the method further comprises:

> storing, in a target area, a quality score string with a length less than the threshold of a string length among the plurality of quality score strings; and
> compressing the quality score string in the target area based on a general-purpose compression algorithm.

11. The method according to any one of claims 1 to 10, wherein the description information compression parameter comprises a partitioning strategy and a second compression model, and compressing the first description information in each of the plurality of blocks based on the description information compression parameter comprises:

> partitioning first description information in any one of the plurality of blocks according to the partitioning strategy to obtain a plurality of description segments; and
> for any one of the plurality of description segments, performing the following steps based on the second compression model:
>
> > if the any description segment is integer-type data, converting the any description segment into a binary format and then compressing the any description segment; or
> > if the any description segment is not integer-type data, compressing the any description segment by using an LZ algorithm; and
> > obtaining, based on compression results corresponding to the plurality of description segments, the description information compression result corresponding to the any block.

12. The method according to any one of claims 1 to 11, wherein the sequence compression parameter comprises a packing length and a third compression model, and compressing the first sequence in each of the plurality of blocks based on the sequence compression parameter comprises:

> processing, according to an N-pack strategy, a first sequence in any one of the plurality of blocks to obtain a fourth sequence, wherein N is an integer greater than 1, and N matches the packing length; and
> compressing the fourth sequence based on the third compression model to obtain the sequence compression result corresponding to the any block.

13. The method according to any one of claims 1 to 12, wherein

> the description information compression result comprises a size of compressed description information and a first compressed stream corresponding to description information, wherein the first compressed stream comprises data obtained by compressing the description information;
> the sequence compression result comprises a size of a compressed sequence and a second compressed stream corresponding to a sequence, wherein the second compressed stream comprises data obtained by compressing the sequence; and
> the quality score compression result comprises a size of a compressed quality score and a third compressed stream corresponding to a quality score, wherein the third compressed stream comprises data obtained by compressing the quality score.

14. The method according to any one of claims 1 to 13, wherein the file header further comprises at least one of the following fields: a format signature, a file size, a third-line status, or a check bit.

15. The method according to any one of claims 1 to 14, wherein the second file further comprises a directory block, and the target block indicates a sequence number of the $1^{st}$ data entry in a block, a position of the block in the second file, and a position of the block in the first file.

16. The method according to any one of claims 1 to 15, wherein the first file is a FASTQ file.

17. The method according to any one of claims 1 to 15, wherein the method further comprises:
decompressing, based on the file header of the second file, a plurality of data blocks in the second file to obtain the first file.

18. A data processing method, comprising:

obtaining, based on a preceding-context model, feature values of a plurality of characters comprised in first data, wherein a feature value of any one of the plurality of characters represents a feature of a preceding-context string, with a preset length, of the any character;
determining a target compression model based on the feature value of the any character; and
compressing the any character based on the target compression model to obtain second data, wherein the second data is a compression result of the first data.

19. The method according to claim 18, wherein obtaining, based on the preceding-context model, the feature values of the plurality of characters comprised in the first data comprises:

for any one of the plurality of characters, obtaining a preceding-context string, with a preset length, of the any character; and
determining a feature value of the any character based on a length of each compressed preceding-context character in the preceding-context string, each preceding-context character in the preceding-context string, and a preset calculation rule.

20. The method according to claim 18 or 19, wherein
determining the target compression model based on the feature value of the any character comprises:

sorting a plurality of characters in the first data based on the feature value to obtain a plurality of strings, wherein each of the plurality of strings corresponds to a same feature value; and
for the $1^{st}$ one of the plurality of strings, determining a first compression model based on a mapping relationship and a feature value corresponding to the $1^{st}$ string, wherein the mapping relationship comprises a correspondence between a plurality of groups of feature values and compression models; and
compressing the any character based on the target compression model to obtain the second data comprises:
compressing the $1^{st}$ string based on the first compression model to obtain third data, wherein the second data comprises the third data.

21. The method according to any one of claims 18 to 20, wherein the first data is a quality score in any block obtained by dividing a FASTQ file into blocks.

22. A communication apparatus, wherein the communication apparatus comprises a processing unit, wherein the processing unit is configured to perform the method according to any one of claims 1 to 21.

23. A communication device, wherein the communication device comprises a processor and a memory, wherein the processor is configured to execute instructions stored in the memory, to enable the communication device to perform the method according to any one of claims 1 to 21.

24. A storage medium, wherein the storage medium stores instructions, and when the instructions are run on a processor, the method according to any one of claims 1 to 21 is performed.

25. A program product, wherein the program product comprises a program, and when the program is run on a processor, the method according to any one of claims 1 to 21 is performed.

**File obtained by compressing a FASTQ file:**

| |
|---|
| File header |
| Data block 1 |
| ... |
| Data block m |
| Ending block |

FIG. 1

**File header:**

| Signature | fileSize | memID | Fastq-stat | Desc_compress_para |
|---|---|---|---|---|
| Seq_compress_para | QS_compress_para | hCRC | | |

FIG. 2

**Quality score compression parameter:**

| Compressors | modelID | cutSize |
|---|---|---|

FIG. 3

**Data block:**

| bType =1 | CompressedBlockSize | Compressed Record Len | Compressed Desc | Compressed Sequence | Compressed Quality Scores | CRC |
|---|---|---|---|---|---|---|

FIG. 4

**Compressed Quality Scores:**

| inSize | cSize | compressedStream |
|---|---|---|

FIG. 5

**Ending block:**

| bType =0 | 0 | CRC |
|---|---|---|

FIG. 6

**Optional block:**

| bType =2 to 255 | Optional block size | Optional block information | CRC |
|---|---|---|---|

FIG. 7

EP 4 664 308 A1

**Directory area:**

| | | | | |
|---|---|---|---|---|
| **Directory block 1:** | bType =5 | Size of the directory block 1 | Information of the directory block 1<br>(A sequence number of the $1^{st}$ data entry in a data block 1, a position of the data block 1 in a second file, and a position of the data block 1 in a first file) | CRC |
| **Directory block 2:** | bType =5 | Size of the directory block 2 | Information of the directory block 2<br>(A sequence number of the $1^{st}$ data entry in a data block 2, a position of the data block 2 in the second file, and a position of the data block 2 in the first file) | CRC |

...

| | | | | |
|---|---|---|---|---|
| **Directory block m:** | bType =5 | Size of the directory block m | Information of the directory block m<br>(A sequence number of the $1^{st}$ data entry in a data block m, a position of the data block m in the second file, and a position of the data block m in the first file) | CRC |

FIG. 8

**Method 100**                                                                S101

Obtain a file header, where the file header includes block information, a description information compression parameter, a sequence compression parameter, and a quality score compression parameter

S102

Obtain a plurality of blocks based on the block information and a first file, where each of the plurality of blocks includes first description information, a first sequence, and a first quality score

S103

Obtain, based on the file header and the plurality of blocks, a data block corresponding to each of the plurality of blocks

| S1031 | S1032 | S1033 |
|---|---|---|
| Compress the first description information in each of the plurality of blocks based on the description information compression parameter, to obtain a description information compression result corresponding to each of the plurality of blocks | Compress the first sequence in each of the plurality of blocks based on the sequence compression parameter, to obtain a sequence compression result corresponding to each of the plurality of blocks | Compress the first quality score in each of the plurality of blocks based on the quality score compression parameter, to obtain a quality score compression result corresponding to each of the plurality of blocks |

S104

Obtain a second file, where the second file is a file obtained by compressing the first file, and the second file includes the file header and the data block corresponding to each of the plurality of blocks

FIG. 9

**S1031:**

S1031a

S1031a: Partition first description information in any one of the plurality of blocks according to a partitioning strategy to obtain a plurality of description segments

S1031b

For any one of the plurality of description segments, perform the following steps based on a second compression model:

S1031c

S1031c: If the any description segment is integer-type data, convert the any description segment into a binary format and then compress the any description segment

S1031d

If the any description segment is not integer-type data, compress the any description segment by using an LZ algorithm

S1031e

Obtain, based on compression results corresponding to the plurality of description segments, a description information compression result corresponding to the any block

FIG. 10

**Method 200**

S201

Obtain, based on a preceding-context model, feature values of a plurality of characters included in first data, where a feature value of any one of the plurality of characters represents a feature of a preceding-context string, with a preset length, of the any character

S202

Determine a target compression model based on the feature value of the any character

S203

Compress the any character based on the target compression model to obtain second data, where the second data is a compression result of the first data

FIG. 11

1200

**Communication apparatus**

1201

First obtaining unit

1202

Second obtaining unit

1204

Third obtaining unit

1203

First compression unit

FIG. 12

1300

**Communication apparatus**

1301

First obtaining unit

1302

Determining unit

1303

Second obtaining unit

FIG. 13

FIG. 14

## INTERNATIONAL SEARCH REPORT

| | International application No. |
|---|---|
| | **PCT/CN2024/072222** |

**A. CLASSIFICATION OF SUBJECT MATTER**

G06F 16/174(2019.01)i

According to International Patent Classification (IPC) or to both national classification and IPC

**B. FIELDS SEARCHED**

Minimum documentation searched (classification system followed by classification symbols)

IPC: G06F

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 数据, 块, 文件, 头, 描述, 质量, 序列, 压缩, 上文, 模型, 特征值, 字符串, data, FASTQ, block, file, head, description, quality, sequence, compress, model, characteristic, feature, string

**C. DOCUMENTS CONSIDERED TO BE RELEVANT**

| Category* | Citation of document, with indication, where appropriate, of the relevant passages | Relevant to claim No. |
|---|---|---|
| A | CN 111628779 A (SHENZHEN BGI LIFE SCIENCES RESEARCH INSTITUTE) 04 September 2020 (2020-09-04) claims 1-10, and description, paragraphs [0034]-[0062] | 1-25 |
| A | CN 112817926 A (BEIJING DBSEC TECHNOLOGY CO., LTD.) 18 May 2021 (2021-05-18) entire document | 1-25 |
| A | US 2018365260 A1 (HUAWEI TECHNOLOGIES CO., LTD.) 20 December 2018 (2018-12-20) entire document | 1-25 |
| A | CN 115699190 A (KONINKL PHILIPS NV) 03 February 2023 (2023-02-03) entire document | 1-25 |
| A | CN 113268459 A (JIANGSU TUOYOU INFORMATION INTELLIGENT TECHNOLOGY RESEARCH INSTITUTE CO., LTD.) 17 August 2021 (2021-08-17) entire document | 1-25 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 March 2024** | **27 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/072222**

| Patent document cited in search report | | | Publication date (day/month/year) | Patent family member(s) | | | Publication date (day/month/year) |
|---|---|---|---|---|---|---|---|
| CN | 111628779 | A | 04 September 2020 | None | | | |
| CN | 112817926 | A | 18 May 2021 | None | | | |
| US | 2018365260 | A1 | 20 December 2018 | KR | 20180086484 | A | 31 July 2018 |
| | | | | KR | 102219745 | B1 | 23 February 2021 |
| | | | | WO | 2018039983 | A1 | 08 March 2018 |
| | | | | US | 11360940 | B2 | 14 June 2022 |
| | | | | EP | 3367275 | A1 | 29 August 2018 |
| CN | 115699190 | A | 03 February 2023 | US | 2023207069 | A1 | 29 June 2023 |
| | | | | JP | 2023522849 | A | 01 June 2023 |
| | | | | WO | 2021204616 | A1 | 14 October 2021 |
| | | | | EP | 4133492 | A1 | 15 February 2023 |
| | | | | BR | 112022020116 | A2 | 20 December 2022 |
| CN | 113268459 | A | 17 August 2021 | None | | | |

Form PCT/ISA/210 (patent family annex) (July 2022)

**EP 4 664 308 A1**

**REFERENCES CITED IN THE DESCRIPTION**

**Patent documents cited in the description**

- CN 202310303465